# EUROPEAN PATENT SPECIFICATION

(11) **EP 3 366 241 B1**
(45) Date of publication and mention of the grant of the patent: **07.09.2022**
(21) Application number: 18155341.3
(22) Date of filing: 06.02.2018
(51) Int. Cl.: A61B 17/70

(54) **SPINAL ROD**
WIRBELSÄULENSTAB
TIGE SPINALE

(30) Priority: 24.02.2017 TW 106106559
(43) Date of publication of application: 29.08.2018
(73) Proprietor: BAUI Biotech Co., Ltd., New Taipei City (TW)
(72) Inventor: CHIEN-YU, Lin, New Taipei City (TW); KUO-WEI, Tseng, New Taipei City, (TW)
(74) Representative: Zaboliene, Reda

(56) References cited:
- WO-A1-2011/158985
- US-A1- 2008 125 787
- US-A1- 2008 262 554
- US-A1- 2009 030 465

## Description

### FIELD

The present disclosure generally relates to a system for stabilization of the spine, and more particularly to a semi-rigid rod assembly for stabilization of the spine.

### BACKGROUND

The spinal rods can be divided into rigid, soft or semi-rigid spinal rods by the flexibility provided by the spinal rods after being implanted. Rigid spinal rods lack flexibility, and are unable to share the stress between the pedicle screw and the vertebrae. After the implantation of the spinal rod, the patient may experience unnatural postures because of the immovability of the rigid spinal rod. The rigid spinal rod is also disadvantageous for the healing of vertebrae due to its' immovability. Commercially available spinal rods are mostly flexible to a certain extent after the implantation, and they could be divided into soft or semi-rigid spinal rods.

Among the commercially available spinal rods, Graft Ligament and Dynesys are two of the soft spinal rods. Graft Ligament is a polyester band structure, and it is connected to pedicle screws for stabilization of the vertebrae. However, Graft Ligament provides poor stabilization of the vertebrae. Dynesys is composed of a PET material core covered by a PCU material, and it is connected to titanium pedicle screws for stabilization of the vertebrae without compromising the flexion and extension of the patient. AccuFlex, Isobar TTL and CD-Horizon Legacy PEEK rod are some of the semi-rigid spinal rods, wherein AccuFlex is a titanium alloy spinal rod connected to the pedicle screws. The AccuFlex is carved with helical structures, the helical structure provides flexibility required for the spinal rod to be compressed or extended. Isobar TTL is composed of titanium and titanium alloy, and it is connected to pedicle screws. The spinal rod can be bend or extended toward the sagittal plane and the frontal plane for 4.5_{°}. CD-Horizon Legacy PEEK rod is composed of PEEK (polyetheretherketone), and it is softer than the titanium spinal rods.

US 2008/0125787 A1 provides a dynamic rod assembly for intervertebral stabilization. The dynamic rod assembly comprises a ball bearing, two ball seats for receiving the ball bearing, spring washers, and springs coupled to the ball seats. The ball seats are coupled to a lower rod and a second rod, respectively. The springs and spring washers enable a longitudinal compression or a lateral movement for the dynamic rod assembly.

US 2008/0262554 A1 provides a dynamic rod for stabilization of the spine. The dynamic rod comprises a first rod portion, a second rod portion, a bias element, and a second bias element. A displacement of the longitudinal axis and a compression of the longitudinal axis for the first rod portion of the dynamic rod are provided by the bias element or the second bias element.

WO 2011/158985 provides a rod for spinal correction. The rod comprises a rear load, a front rod, a first elastic member, and a second elastic member. A stretching in length or a twisting of the rod can be provided by the first elastic member and the second elastic member.

The spring or elastic elements in the '787 patent, the '554 patent, and the '985 patent are not housed by an upper or a lower rod of the rod, therefore the elasticity or flexibility provided by the spring or elastic elements are not sufficiently passed onto the upper or the lower rod. Commercially available spinal rods have higher flexibility, but they tend to provide poor stabilization of the vertebrae. The semi-rigid spinal rod systems may provide better stabilization of the vertebrae, but they have lower flexibility. Some commercially available spinal rods adopt helical designs or spring in the central section of the spinal rod; however, these structures may lead to the concentration of stress in specific parts of the spinal rods, which may lead to the fracture of the spinal rod.

### BRIEF DESCRIPTION OF THE DRAWINGS

The present description will be better understood from the following detailed description read in light of the accompanying drawings, where:
FIG. 1 is a perspective view of the spinal rod in accordance with an embodiment of the present disclosure.
FIG. 2 is an exploded view of the spinal rod of FIG. 1 in accordance with an embodiment of the present disclosure.
FIG. 3 is a perspective view of structural elements of the spinal rod of FIG. 1 in accordance with an embodiment of the present disclosure.
FIG. 4 is an appearance of the movement and rotation of the spinal rod of FIG. 1 in the frontal plane in accordance with an embodiment of the present disclosure.
FIG. 5 is an appearance of the movement and rotation of the spinal rod of FIG. 1 in the sagittal plane in accordance with an embodiment of the present disclosure.
FIG. 6 is an appearance of compressing the spinal rod of FIG. 1 in accordance with an embodiment of the present disclosure.
FIG. 7 is an appearance of the spinal rod in an embodiment of the spinal stabilization system in accordance with an embodiment of the present disclosure.
FIG. 8 is an appearance of structural elements of the spinal stabilization system of FIG. 7 in accordance with an embodiment of the present disclosure.

### DETAILED DESCRIPTION OF THE PREFERRED EMBODIMENTS

The present disclosure is directed to a spinal rod for higher flexibility and better stabilization for the spine.

The present disclosure is directed to a spinal rod, comprising: a rod assembly comprising a movable rod, a stationary rod and a cap, wherein the movable rod is on an upper side of the spinal rod and further comprises a second connecting portion and a second rod portion on the upper side of the second connecting portion, a diameter of the second connecting portion is larger than a diameter of the second rod portion, the stationary rod is on a lower side of the spinal rod and further comprises a first connecting portion and a first rod portion on the lower side of the first connecting portion, a diameter of the first connecting portion is larger than a diameter of the first rod portion, and the cap is between the stationary rod and the movable rod for fixing the movable rod and the stationary rod; and an elastic assembly comprising a first elastic element and a second elastic element, wherein the elastic assembly is accommodated by the rod assembly, the first elastic element is between the cap and the stationary rod, a compression of the first elastic element by the movable rod shortens an overall length of the rod assembly, and the second elastic element provides a flexibility for movements of the movable rod and the stationary rod in a frontal plane and a sagittal plane,the spinal rod being characterized in that: the second elastic element is a rod structure partially accommodated by the movable rod and the stationary rod and passes through a center of the first elastic element, an end of the second elastic element is fixed inside the first rod portion, another end of the second elastic element is fixed inside the second rod portion, and the first rod portion, first connecting portion, the second rod portion, and the second connecting portion are of hollow structures.

In one embodiment, each of the first connecting portion of the stationary rod and the cap comprises a screw thread structure for connecting the cap and the first connecting portion.

In one embodiment, the second connecting portion passes through and is sleeved by the cap. The second connecting portion is contacted with the first elastic element, wherein one end of the second elastic element passes through the first elastic element and is fixed inside the second rod portion.

In one embodiment, the compression of the first elastic element by the movable rod shortens an overall length of the rod assembly, the shortened length is ranged from 0 mm to 1 mm.

In one embodiment, the flexibility for movements of the movable rod and the stationary rod in the frontal plane and the sagittal plane provided by the second elastic element defined by a pivot angle of 0° to 20° .

In one embodiment, the movable rod and the stationary rod rotate about an axis for 360°.

In one embodiment, the first elastic element can be selected from one or more of a helical spring, a flat spring or an elastomeric silicon object.

In one embodiment, the rod assembly, the first elastic element and the second elastic element can be comprised of titanium alloy, nickel-titanium alloy, vitallium or stainless steel.

The present disclosure is also directed to a spinal stabilization system, comprising a plurality of pedicle screws, a plurality of nuts and the spinal rod. Each of the pedicle screws comprises a rod-receiving slot, wherein the spinal rod is fixed on the rod-receiving slot by the nuts.

The present disclosure is directed to a spinal rod designed with elastic elements. The elastic elements reduce the stress-shielding effect caused by the conventional spinal rod and increase the spine fusion rate. Comparing to spinal rod composed of polymers, the spinal rod provided by the present disclosure not only provides the flexibility needed for the rod assembly to move in the frontal plane and the sagittal plane, but also the compressibility needed for the rod assembly to be compressed axially. The spinal rod provided by the present disclosure has a better adaptation to physical movements of the spine, and the quality of life of the patient receiving the spinal stabilization system implantation is increased. The spinal rod provided by the present disclosure has a better stabilization and relocation effect for the spine.

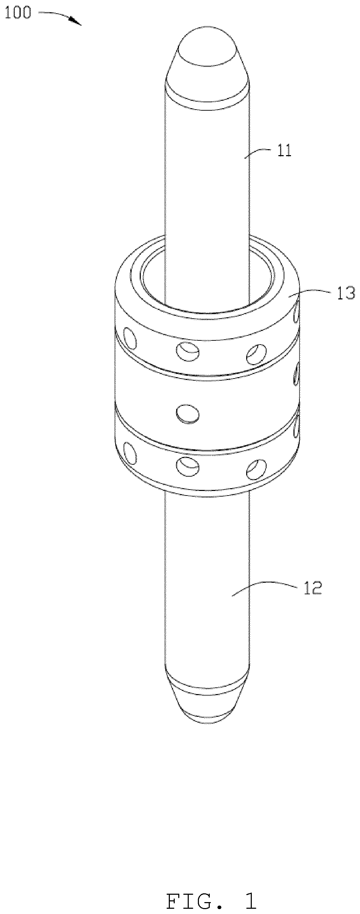

FIG.1 is a perspective view of a spinal rod in accordance with an embodiment of the present disclosure. In the embodiment, the spinal rod 100 comprises a rod assembly, the rod assembly is aligned axially within the spinal rod. The rod assembly comprises a stationary rod 12, a movable rod 11 and a cap 13. In the embodiment, the movable rod 11 is located on the upper side of the spinal rod, namely a side that is closer to the patient's head when the spinal rod 100 is implanted. The stationary rod 12 is located on the lower side of the spinal rod, namely a side that is closer to the patient's hip when the spinal rod 100 is implanted. A longitudinal axis extending from a lower end of the stationary rod 12 to the upper end of the movable rod 11 is the axis of the spinal rod 100. The axis is parallel to a direction from the patient's hip to the patient's head. The cap 13 is located between the stationary rod 12 and the movable rod 11, and is used to connect the movable rod 11 and the stationary rod 12.

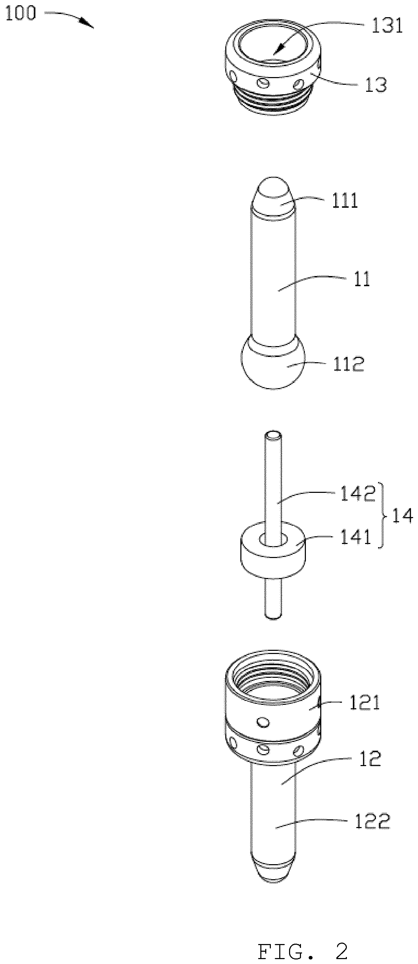

Referring to FIG. 2, the spinal rod 100 further comprises an elastic assembly 14, the elastic assembly 14 can be accommodated by the rod assembly and shall not have direct contact with the patient's tissue. The elastic assembly 14 comprises a first elastic element 141 and a second elastic element 142. The first elastic element 141 is between the cap 13 and the stationary rod 12, a compression of the first elastic element 141 by the movable rod 11 shortens an overall length of the rod assembly. The second elastic element 142 passes through the first elastic element 141 and is partially accommodated by the movable rod 11 and the stationary rod 12 to provide a flexibility for the movements of the movable rod 11 in the frontal plane and the sagittal plane. In the present embodiment, the first elastic element 141 can be selected from one or more of a helical spring, a flat spring or an elastic silicone object. The second elastic element 142 can be selected from one or more of a linear spring, a carved spring or a linear flat spring. It is to be noted that the first elastic element 141 and the second elastic element 142 are not limited to above structures in accordance with embodiments of the present disclosure.

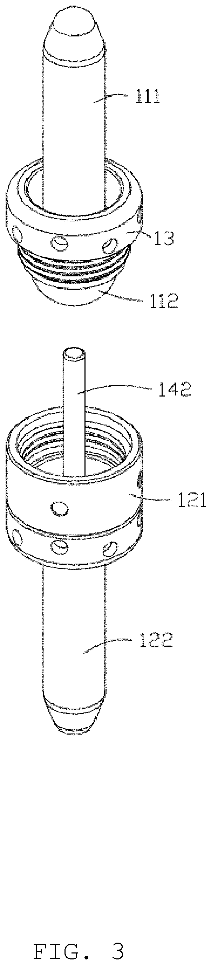

In FIG. 2 and FIG. 3, the stationary rod 12 comprises a first connecting portion 121 and a first rod portion 122. A diameter of the first connecting portion 121 is larger than a diameter of the first rod portion 122. The first rod portion 122 and the first connecting portion 121 are of hollow structures. One end of the second elastic element 142 is fixed inside the first rod portion 122, and the first connecting portion 121 houses the first elastic element 141. Each of the first connecting portion 121 of the stationary rod 12 and the cap 13 comprises screw thread structures, and the first connecting portion 121 is connected to the cap 13. The movable rod 11 comprises a second connecting portion 112 and a second rod portion 111. A diameter of the second connecting portion 112 is larger than a second rod portion 111. The second rod portion 111 and the second connecting portion 112 are of hollow structures. The cap 13 comprises an opening 131. As illustrated by FIG. 3, the second connecting portion 112 may pass through the opening 131 and being sleeved by the cap 13, and the second connecting portion 112 may be contacted with the first elastic element 141. The bottom of the second connecting portion 112 may comprise an aperture (not shown) for the insertion of the second elastic element 142. An upper end of the second elastic element 142 may pass through the first elastic element 141, and then enter the second connecting portion 112 via the aperture of the second connecting portion 112, and be fixed inside the second rod portion 111. In the present embodiment, the second connecting portion 112 is a partially spherical structure, whereas in other embodiments the structure of the second connecting portion 112 is not limited to spherical structure. The diameter of the second connecting portion 112 may be the largest circumference of the spherical structure. In the present embodiment, the second elastic element 142 is located centrally at axes of the movable rod 11 and the stationary rod 12.

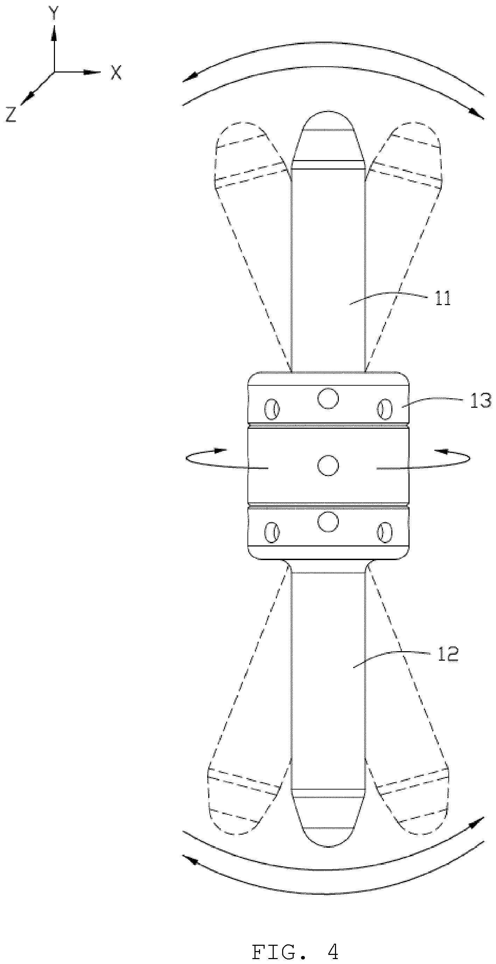

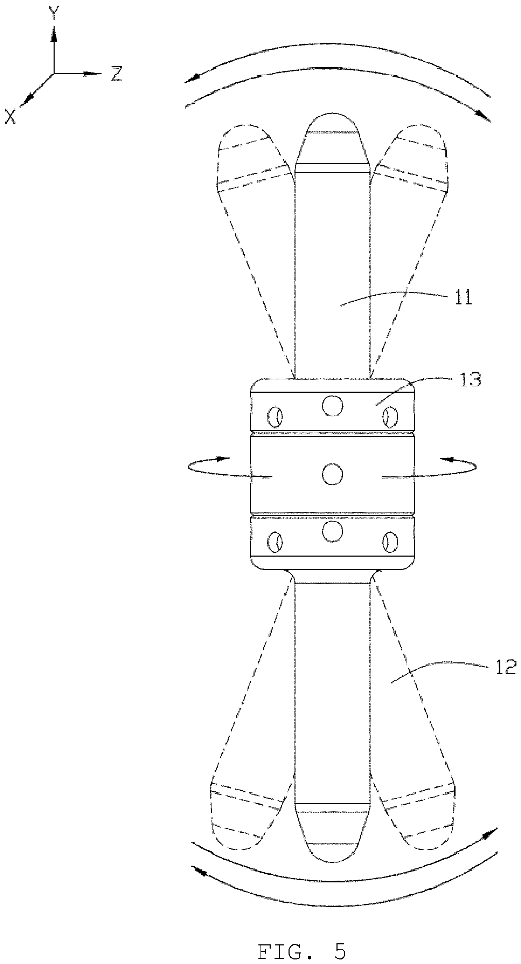

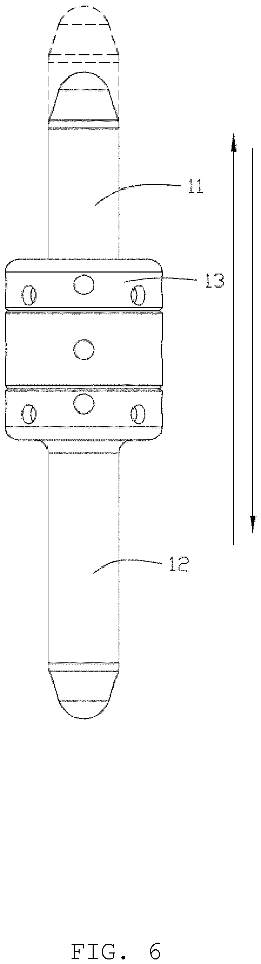

As illustrated by FIG. 4, FIG. 5 and FIG. 6, the elastic assembly 14 provides the rod assembly the compressibility for axial compression, the flexibility for rotating and moving in the sagittal plane and the frontal plane. The flexion or extension in the sagittal plane and lateral bending in the frontal plane for the patient after implanted with the spinal rod 100 is possible. Specifically, the second elastic element 142 is inside the movable rod 11 and the stationary rod 12 to provide the flexibility needed for the movable rod 11 and the stationary rod 12 to be moved or to be rotated in the sagittal plane and the frontal plane. FIG. 4 is the movement of the spinal rod 100 in the frontal plane, wherein the frontal plane of the spinal rod 100 is the X-Y plane of FIG. 4, or a longitudinal cross section that divides the spinal rod 100 into a front part and a rear part; FIG. 5 is the movement of the spinal rod 100 in the sagittal plane, wherein the sagittal plane of the spinal rod 100 is the Y-Z plane of FIG. 5, or a longitudinal cross section that divides the spinal rod 100 into a right part and a left part. In FIG. 4 and FIG. 5, the spinal rod 100 may have a pivot angle of 0_{°} to 20_{°} in the frontal plane for lateral bending and in the sagittal plane for flexion and extension. The spinal rod 100 can also being rotated about an axis for 360_{°}, as rotation is a combination of movements in both the frontal plane and the sagittal plane. In FIG. 6, the overall length of the rod assembly is shortened by the compression of the first elastic element 141. The first elastic element 141 is between the cap 13 and the stationary rod 12, the first elastic element 141 is compressed by the movable rod 11 to compress the rod assembly axially, therefore the overall length of the rod assembly is shortened by about 1 mm.

In one embodiment, the rod assembly, the first elastic element 141 and the second elastic element 142 can be comprised of titanium alloy, nickel-titanium alloy, vitallium or stainless steel. The rod assembly, the first elastic element 141 and the second elastic element 142 can be comprised of the same or different materials. In other embodiments, the rod assembly, the first elastic element 141 and the second elastic element 142 can be comprised of other materials other than above.

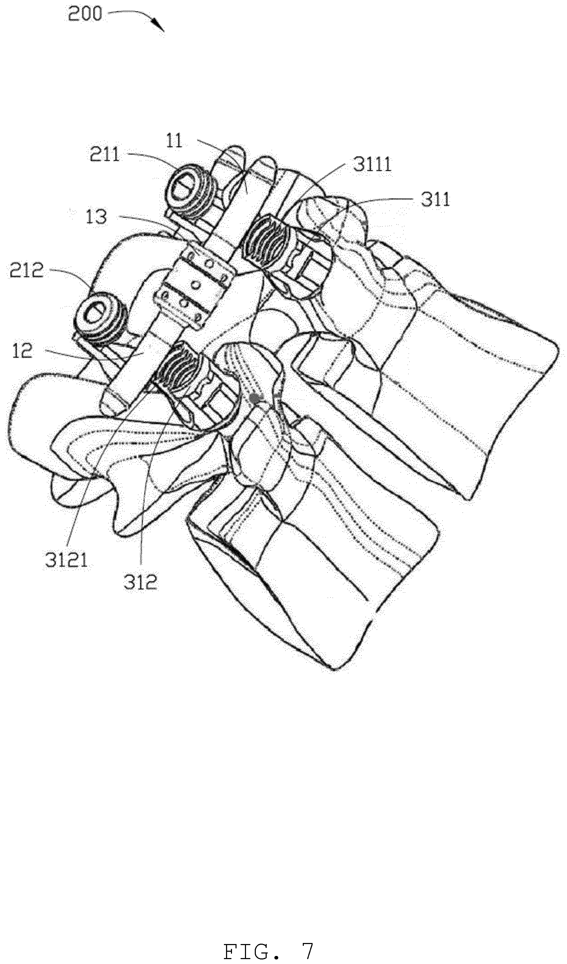

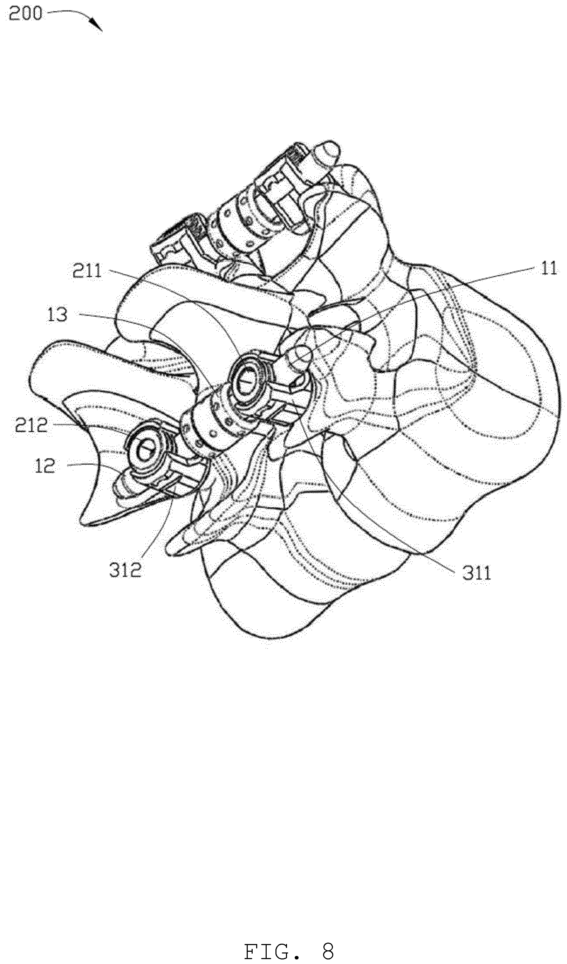

The present disclosure further provides an embodiment of a spinal stabilization system 200 applied with the above spinal rod 100. As illustrated by FIG.7 and FIG.8, the spinal stabilization system 200 comprises pedicle screws 311, 312 and nuts 211, 212. Each of the pedicle screws 311, 312 comprises a rod-receiving slot 3111, 3121. The combination of the spinal rod 100 and the pedicle screws 311, 312 has an identical configuration with conventional spinal rods and pedicle screws, namely the spinal rod 100 is fixed on the rod-receiving slot 3111, 3121 of the pedicle screw 311, 312 via the nuts 211, 212. The above configuration stabilizes the pedicle screws 311, 312 on adjacent vertebrae. In the present embodiment, the spinal stabilization system 200 can be implanted on L1 to L5 of the lumbar spine. In other embodiments, the spinal stabilization system 200 can be implanted on other parts of the spine. When implementing above configuration, the movable rod 11 is on the upper side of the spinal rod 100, namely a side toward the head of the patient when implanting the spinal rod 100; the stationary rod 12 is on the lower side of the spinal rod 100, namely a side toward the hip of the patient when implanting the spinal rod 100.

The spinal stabilization system 200 is capable to relocate the dislocated vertebrae caused by fracture of dislocation of the bone, in order to reduce the suppression to the neural tissues from the dislocated vertebrae. Because the spinal rod 100 comprises an elastic assembly 14 to provide the compressibility for axial compression of the rod assembly and the flexibility for movements in the sagittal plane and the frontal plane, the patient's spine is able to move within a range of limited angles in the lateral or the anteroposterior direction when implanted with the spinal stabilization system 200 using the spinal rod 100 provided by the present disclosure, such as mild extension or flexion; the present disclosure greatly increases the quality of life for the patient implanted with the spinal stabilization system 200.

The foregoing descriptions of specific compositions of the present disclosure have been presented for purposes of illustration and description. They are not intended to be exhaustive or to limit the disclosure to the precise compositions disclosed and obviously many modifications and variations are possible in light of the above teaching. The examples were chosen and described in order to best explain the principles of the disclosure and its practical application, to thereby enable others skilled in the art to best utilize the disclosure with various modifications as are suited to the particular use contemplated. It is intended that the scope of the disclosure be defined by the claims appended hereto and their equivalents.

## Claims

1. A spinal rod (100), comprising:
a rod assembly comprising a movable rod (11), a stationary rod (12) and a cap (13), wherein the movable rod (11) is on an upper side of the spinal rod (100) and further comprises a second connecting portion (112) and a second rod portion (111) on the upper side of the second connecting portion (112), a diameter of the second connecting portion (112) is larger than a diameter of the second rod portion (111), the stationary rod (12) is on a lower side of the spinal rod (100) and further comprises a first connecting portion (121) and a first rod portion (122) on the lower side of the first connecting portion (121), a diameter of the first connecting portion (121) is larger than a diameter of the first rod portion (122), and the cap (13) is between the stationary rod (12) and the movable rod (11) for fixing the movable rod (11) and the stationary rod (12); and
an elastic assembly (14) comprising a first elastic element (141) and a second elastic element (142), wherein the elastic assembly (14) is accommodated by the rod assembly, the first elastic element (141) is between the cap (13) and the stationary rod (12), a compression of the first elastic element (141) by the movable rod (11) shortens an overall length of the rod assembly, and the second elastic element (142) provides a flexibility for movements of the movable rod (11) and the stationary rod (12) in a frontal plane and a sagittal plane,
the spinal rod being **characterized in that**:
the second elastic element (142) is a rod structure partially accommodated by the movable rod (11) and the stationary rod (12) and passes through a center of the first elastic element (141), an end of the second elastic element (142) is fixed inside the first rod portion (122), another end of the second elastic element (142) is fixed inside the second rod portion (111), and the first rod portion (122), first connecting portion (121), the second rod portion (111), and the second connecting portion (112) are of hollow structures.

2. The spinal rod (100) of claim 1, wherein the cap (13) and the stationary rod (12) further comprises a screw thread structure for connecting the cap (13) and the stationary rod (12).

3. The spinal rod (100) of claim 1, wherein
the first elastic element (141) is accommodated by the first connecting portion (121).

4. The spinal rod (100) of claim 1, 2 or 3, wherein
the second connecting portion (112) passes through and is sleeved by the cap (13), and the second connecting portion (112) is contacted with the first elastic element (141).

5. The spinal rod (100) of claim 1, 2 or 3, wherein the shortened overall length of the rod assembly is ranged from 0 mm to 1 mm.

6. The spinal rod (100) of claim 1, 2 or 3, wherein the flexibility for the movements of the movable rod (11) and the stationary rod (12) in the frontal plane and the sagittal plane provided by the second elastic element (142) is defined by a pivot angle of 0° to 20°.

7. The spinal rod (100) of claim 1, 2 or 3, wherein the movable rod (11) and the stationary rod (12) rotate about an axis for 360°.

8. The spinal rod (100) of claim 1, 2 or 3, wherein the first elastic element (141) is selected from one or more of a helical spring, a flat spring or an elastomeric silicon object.

9. The spinal rod (100) of claim 1, 2 or 3, wherein the rod assembly, the first elastic element (141) and the second elastic element (142) can be made of titanium alloy, nickel-titanium alloy, vitallium, or stainless steel.

10. A spinal stabilization system (200), comprising:
a plurality of nuts (211, 212);
a plurality of pedicle screws (311, 312), each of the pedicles screws (311, 312) comprising a rod-receiving slot (3111, 3121); and
a spinal rod (100) of claim 1, 2 or 3,
wherein the spinal rod (100) is fixed on the rod-receiving slot (3111, 3121) by the nuts (211, 212).

## Patentansprüche

1. Wirbelsäulenstab (100), umfassend:
eine Stangenanordnung, die eine bewegliche Stange (11), eine stationäre Stange (12) und eine Kappe (13) umfasst, wobei sich die bewegliche Stange (11) auf einer oberen Seite der Wirbelsäulenstange (100) befindet und ferner den zweiten Verbindungsabschnitt umfasst (112) und dem zweiten Stangenabschnitt (111) auf der oberen Seite des zweiten Verbindungsabschnitts (112), einem Durchmesser des zweiten Verbindungsabschnitts (112) größer als ein Durchmesser des zweiten Stangenabschnitts (111) ist,
befindet sich die stationäre Stange (12) auf einer unteren Seite der Wirbelsäulenstange (100) und umfasst ferner den ersten Verbindungsabschnitt(121) und den ersten Stangenabschnitt (122) auf der unteren Seite des ersten Verbindungsabschnitts (121), ein Durchmesser des ersten Verbindungsabschnitts (121) größer als ein Durchmesser des ersten Stangenabschnitts (122) ist, und die Kappe (13), die sich zwischen der stationären Stange (12) und der beweglichen Stange (11) befindet, um die bewegliche Stange (11) und
die stationäre Stange (12) zu befestigen; und
eine elastische Anordnung (14), die ein erstes elastisches Element (141) und ein zweites elastisches Element (142) umfasst, wobei die elastische Anordnung (14) von der Stangenanordnung aufgenommen wird, das erste elastische Element (141) zwischen der Kappe (13) und der stationären Stange (12) liegt,
ein Zusammendrücken des ersten elastischen Elements (141) durch die bewegliche Stange (11) eine Gesamtlänge der Stangenanordnung verkürzt, und das das zweite elastische Element (142) eine Flexibilität für Bewegungen der beweglichen Stange (11) und der stationären Stange (12) in einer Frontalebene und einer Sagittalebene bereitstellt,
wobei der Wirbelsäulenstab **dadurch gekennzeichnet ist, dass**:
das zweite elastische Element (142) eine Stangenstruktur ist, die teilweise von der beweglichen Stange (11) und der stationären Stange (12) aufgenommen wird und durch eine Mitte des ersten elastischen Elements (141), ein Ende des zweiten elastischen Elements (142) verläuft) innerhalb des ersten Stangenabschnitts (122) befestigt ist, ein anderes Ende des zweiten elastischen Elements (142) innerhalb des zweiten Stangenabschnitts (111) befestigt ist, und der erste Stangenabschnitt (122), der erste Verbindungsabschnitt (121), der der zweite Stangenabschnitt (111) und der zweite Verbindungsabschnitt (112) hohle Strukturen aufweisen.

2. Wirbelsäulenstab (100) nach Anspruch 1, wobei die Kappe (13) und der stationäre Stab (12) ferner eine Schraubgewindestruktur zum Verbinden der Kappe (13) und des stationären Stabs (12) aufweisen.

3. Wirbelsäulenstab (100) nach Anspruch 1, wobei
das erste elastische Element (141) von dem ersten Verbindungsabschnitt (121) aufgenommen wird.

4. Wirbelsäulenstab (100) nach Anspruch 1, 2 oder 3, wobei
der zweite Verbindungsabschnitt (112) durch die Kappe (13) hindurchgeht und von dieser umhüllt ist, und der zweite Verbindungsabschnitt (112) mit dem ersten elastischen Element (141) in Kontakt ist.

5. Wirbelsäulenstab (100) nach Anspruch 1, 2 oder 3, wobei die verkürzte Gesamtlänge der Stabanordnung im Bereich von 0 mm bis 1 mm liegt.

6. Spinalstange (100) nach Anspruch 1, 2 oder 3, wobei die Flexibilität für die Bewegungen der beweglichen Stange (11) und der stationären Stange (12) in der Frontalebene und der Sagittalebene durch das zweite elastische Element bereitgestellt wird (142) ist durch einen Schwenkwinkel von 0° bis 20° definiert.

7. Wirbelsäulenstab (100) nach Anspruch 1, 2 oder 3, wobei sich der bewegliche Stab (11) und der stationäre Stab (12) um 360° um eine Achse drehen.

8. Wirbelsäulenstab (100) nach Anspruch 1, 2 oder 3, wobei das erste elastische Element (141) aus einem oder mehreren von einer Schraubenfeder, einer flachen Feder oder einem Elastomeren Silikonobjekt ausgewählt ist.

9. Wirbelsäulenstab (100) nach Anspruch 1, 2 oder 3, wobei die Stabanordnung, das erste elastische Element (141) und das zweite elastische Element (142) aus Titanlegierung, Nickel-Titan-Legierung, Vitallium oder Edelstahl.

10. Wirbelsäulenstabilisierungssystem (200), umfassend: eine Vielzahl von Muttern (211, 212);
eine Vielzahl von Pedikelschrauben (311, 312), wobei jede der Pedikelschrauben (311, 312) einen Stangenaufnahmeschlitz (3111, 3121) umfasst; und
eine Wirbelsäulenstange (100) nach Anspruch 1, 2 oder 3,
wobei der Wirbelsäulenstab (100) an dem Stabaufnahmeschlitz (3111, 3121) durch die Muttern (211, 212) befestigt ist.

## Revendications

1. Une tige vertébrale (100), comprenant :
un ensemble de tige comprenant une tige mobile (11), une tige fixe (12) et un capuchon (13), dans lequel la tige mobile (11) se trouve sur un côté supérieur de la tige vertébrale (100) et comprend en outre une seconde partie de connexion (112) et une seconde partie de tige (111) sur le côté supérieur de la seconde partie de connexion (112), un diamètre de la seconde partie de connexion (112) est plus grand qu'un diamètre de la seconde partie de tige (111), la tige stationnaire (12) se trouve sur un côté inférieur de la tige vertébrale (100) et comprend en outre une première partie d'assemblage (121) et une première partie de tige (122) sur le côté inférieur de la première partie de connexion (121),
un diamètre de la première partie de connexion (121) est plus grand qu'un diamètre de la première partie de tige (122), et le capuchon (13) se trouve entre la tige fixe (12) et la tige mobile (11) pour fixer la tige mobile (11) et la tige fixe (12) ; et
un ensemble élastique (14) comprenant un premier élément élastique (141) et un second élément élastique (142), dans lequel l'ensemble élastique (14) est logé par l'ensemble des tiges,
le premier élément élastique (141) se trouve entre le capuchon (13) et la tige stationnaire (12), une compression du premier élément élastique (141) par la tige mobile (11) raccourcit une longueur totale de l'ensemble des tiges, et le second élément élastique (142) fournit une flexibilité pour les mouvements de la tige mobile (11) et de la tige fixe (12) dans un plan frontal et un plan sagittal,
la tige vertébrale étant **caractérisée par le fait que** :
le second élément élastique (142) est une structure de tige partiellement logée par la tige mobile (11) et la tige fixe (12) et passe par le centre du premier élément élastique (141), une extrémité du second élément élastique (142) est fixée à l'intérieur de la première partie de tige (122), une autre extrémité du second élément élastique (142) est fixée à l'intérieur de la seconde partie de tige (111), et la première partie de tige (122), la première partie de connexion (121), la seconde partie de tige (111) et la seconde partie de connexion (112) sont des structures creuses.

2. La tige vertébrale (100) selon la revendication 1, dans laquelle le capuchon (13) et la tige stationnaire (12) comprennent en outre une structure à filetage de vis pour relier le capuchon (13) et la tige stationnaire (12).

3. La tige vertébrale (100) selon la revendication 1, dans laquelle
le premier élément élastique (141) est logé par la première partie de connexion (121).

4. La tige vertébrale (100) selon les revendications 1, 2 ou 3 dans lesquelles
la seconde partie de connexion (112) passe à travers et est gainée par le capuchon (13), et la seconde partie de connexion (112) est en contact avec le premier élément élastique (141).

5. La tige vertébrale (100) selon les revendications 1, 2 ou 3, dans lesquelles la longueur totale raccourcie de l'ensemble des tiges est comprise entre 0 mm et 1 mm.

6. La tige vertébrale (100) selon les revendications 1, 2 ou 3, dans lesquelles la flexibilité pour les mouvements de la tige mobile (11) et la tige fixe (12) dans le plan frontal et le plan sagittal fourni par le second élément élastique (142) est définie par un angle de pivotement de 0° à 20°.

7. La tige vertébrale (100) selon les revendications 1, 2 ou 3, dans lesquelles la tige mobile (11) et la tige fixe (12) tournent autour d'un axe sur 360°.

8. La tige vertébrale (100) selon les revendications 1, 2 ou 3, dans lesquelles le premier élément élastique (141) est choisi parmi un ou plusieurs ressorts hélicoïdaux, un ressort plat ou un objet en silicone élastomère.

9. .La tige vertébrale (100) selon les revendications 1, 2 ou 3, dans lesquelles l'ensemble des
tiges, le premier élément élastique (141) et le second élément élastique (142) peuvent être constitués d'un alliage de titane, d'un alliage de nickel-titane, de vitallium ou d'acier inoxydable.

10. Un système de stabilisation vertébrale (200), comprenant :
une pluralité d'écrous (211, 212) ;
une pluralité de vis pédiculaires (311, 312), chacune des vis pédiculaires (311, 312) comprenant une fente de réception de tige (3111, 3121) ; et
une tige vertébrale (100) selon les revendications 1, 2 ou 3,
dans lesquelles la tige vertébrale (100) est fixée sur la fente de réception de tige (3111, 3121) par les écrous (211, 212).
